# EUROPEAN PATENT SPECIFICATION

(11) **EP 0 923 350 B1**
(45) Date of publication and mention of the grant of the patent: **30.07.2003**
(21) Application number: 97908750.9
(22) Date of filing: 04.03.1997
(51) Int. Cl.: A61B 19/08

(54) **STERILE SURGICAL COUPLER AND DRAPE**
STERILE CHIRURGISCHE KUPPLUNG UND ABDECKTUCH
COUPLEUR ET ENVELOPPE SOUPLE STERILE A USAGE CHIRURGICAL

(30) Priority: 04.03.1996 US 609954
(43) Date of publication of application: 23.06.1999
(73) Proprietor: Adair, Edwin L., Castle Pines Village Colorado 80104 (US)
(72) Inventor: Adair, Edwin L., Castle Pines Village Colorado 80104 (US)
(74) Representative: Bibby, William Mark
(86) International application number: US9703049
(87) International publication number: WO97032534

(56) References cited:
- EP-A- 0 437 004
- EP-A- 0 591 936
- US-A- 4 522 196
- US-A- 4 844 071
- US-A- 5 205 280
- US-A- 5 591 119
- US-E- R E34 002

## Description

### Technical Field

This invention relates to a sterile surgical coupler and drape for use in the sterile environment of an operating room. More particularly, the invention relates to such a coupler and drape which encloses an unsterile camera setup including a camera and an optical connector wherein the unsterile optical connector attaches to a first end of the coupler and a second end of the coupler in turn attaches to a sterile endoscope.

### Background Art

For many years, unsterile surgical cameras and optical connectors known in the art as "C" or "V" mount connectors have been used in surgery by placing them into a sterile plastic bag or drape that has a distal end including an opening for receiving the eyepiece of a sterile or disinfected endoscope. The act of coupling the sterile or disinfected endoscope through the opening in the drape to the unsterile optical connector can create contamination. That is, the interior of the drape that houses the unsterile camera and optical connector is exposed to the sterile environment of an operating room through the hole located at the distal end of the drape. When the eyepiece of the endoscope is inserted through this hole for connection to the optical connector, this hole often becomes enlarged thus enhancing the possibility of contamination traveling from the interior of the drape to the sterile operating room. The further acts of aligning the endoscope with the optical connector and sealing the distal end of the drape around the protruding distal portion of the endoscope can also result in further contamination.

My earlier U.S. Patent No. Re. 34,002 discloses a sterilizable video camera cover which has a connector including a guideway for receiving an unsterile video camera within it in a predetermined fixed orientation. One end of the video camera cover receives a sterile mount and endoscope in a fixed position with respect to the camera. An accordion-folded sleeve is positioned on the camera cover and is extended over the trailing cables of the camera to maintain the sterile environment within the operating room even though the camera and trailing cables are unsterile.

A sterile pouch for containing a standard still picture camera for use in an operating room is shown in U.S. Patent No. 2,537,303 to Cobb, Jr. et al. However, there is no thought in this device of connecting the camera to other optical means. Other containers for protecting cameras in varying environments are shown in U.S. Patent No. 3,026,784 to Byers, U.S. Patent No. 3,821,759 to Vooght and U.S. Patent No. 2,132,549 to Wenstrom. However, none of these references are intended for use in an operating room to maintain the environment within the operating room in a sterile condition when the camera is not sterile.

There are numerous references which disclose sterile drapes or covers for isolating an unsterile camera and its trailing cables from the sterile environment of an operating room. Examples of such references include U.S. Patent No. 5,274,500 to Dunn, U.S. Patent No. 5,078,483 to Herzberg, U.S. Patent No. 5,198,894 to Hicks, and U.S. Patent No. 5,325,846 to Szabo. While each of these references may be adequate for their intended purpose, none of these references disclose a device which allows endoscopes to be freely interchanged with a single camera setup and yet maintain the required sterility of an operating room.

European Patent Application No. EP 0 437 004 discloses a sheeting cover for the protection from contamination of a surgical instrument, more particularly, an endoscope comprising a tubular sheeting which is delimited by an insertion port and an exit port positioned opposite the insertion port, the exit port having a resilient membrane and a perforation whose outer edges clamp into a frame. The frame spreads the tubular sheeting open. The membrane with the perforation closely rests against the outer contours of the part of the instrument projecting through it so that reliable protection of an enveloped device is prevented from being contaminated.

U.S. Patent No. 5,205,280 discloses a quick release coupling assembly for an endoscopic instrument, the assembly including an ocular mounting surface having an aperture and a longitudinal axis passing through the center of the aperture and the mounting surface. A guide wall is radially spaced from and substantially concentric with the aperture of the ocular mounting surface, and the guide wall partially encircles the aperture to define an alignment recess adjacent the mounting surface for receiving the proximal end of an endoscopic instrument. At least one gripping jaw is provided for axial reciprocation. The jaw comprises an inner tab extending outwardly from the mounting surface, a retainer flange extending radially inwardly, and an engagement surface which may be preferably angularly disposed relative to the axis. In a preferred embodiment, the assembly includes a pair of oppositely disposed gripping jaws for releasably engaging the proximal end of the eyepiece of the endoscope to be coupled. The engagement surface may be tapered to readily accommodate eyepieces of various sizes and shapes.

U.S. Patent No. 4,844,071 discloses a coupler device for coupling an endoscope to a camera head. The coupler includes a housing that defines an enclosed light path extending along an optical axis between end portions of the housing together with mounting components for attaching a first end portion of the housing to an endoscope and a second end portion of the housing to a camera head. A lens mounted within the housing between the first and second end portions magnifies an image from the endoscope to provide a magnified image to the camera head. A quick release arrangement in one embodiment enables attachment of the endoscope by advancing the endoscope axially without having to rotate it while a focusing ring enables fine focusing adjustments. An imaging system includes an imaging endoscope with a quick release connector hub, a coupler device for attachment between the connector hub and a camera head and an interchangeable eyepiece for interfacing the endoscope to a human eye.

### Disclosure of the Invention

An apparatus and method is provided for enclosing a non-sterile video camera, its trailing cables, and a standard optical connector such as a "C" mount connector for use of the camera in the sterile environment of an operating room.

According to the invention there is provided an apparatus for optically coupling a sterile endoscope to a sterile camera setup including an unsterile optical connector mounted to an unsterile camera having trailing cables, and for enclosing the unsterile camera setup in a sterile enclosure for use of the unsterile camera setup in a sterile surgical environment, said apparatus comprising, a sterile coupler having a first end including a first mounting structure for connection to the unsterile optical connector, and having a second end including a second mounting structure for connection to the sterile endoscope, said sterile coupler further having an optical path positioned between said first and second ends thereof through which an image may be transmitted from the sterile endoscope to the unsterile camera, a sterile barrier positionable between said first and second ends of said sterile coupler, said apparatus further including a sterile drape having an open distal end and including a drape ring formed thereon, said sterile drape positionable over said first end of said coupler and having a proximal end extendable over the unsterile optical connector, the unsterile camera and its trailing cables; and means for sealing said distal end of said drape between said first and second ends of said sterile coupler whereby said first end of said coupler is isolated from the sterile surgical environment located outside said drape, said sealing means including and second ends of said sterile coupler whereby said first end of said coupler is isolated from the sterile surgical environment located outside said drape, said sealing means including
a locking ring positionable between said-first end and said second end of said sterile coupler, and engaging said drape ring to compress the drape ring against the coupler between said locking ring and said coupler.

According to the invention there is further provided a method of coupling an unsterile camera setup including an optical connector and camera to a sterile endoscope wherein differing endoscopes may be freely interchanged without compromising the sterility of a surgical environment, said method comprising the steps of providing a sterile coupler having a first end attachable to the unsterile camera setup and a second end attachable to the sterile endoscope, providing a sterile drape having an open distal end including a drape ring formed thereon positionable over the first end of the coupler and having a proximal end extendable over the camera setup inserting the unsterile optical connector mounted to the unsterile camera within the proximal end of the drape, attaching the unsterile optical connector the first end of the coupler, and attaching the sterile endoscope to the second end of the coupler characterized in that said method further comprises the step of sealing the distal end of the drape around the first end of the coupler by a locking ring engageable with the drape ring and said coupler thereby providing a sterile seal wherein the.first end of the coupler is sealed within the drape and the second end of the coupler is exposed to the sterile surgical environment.

The first end of the coupler may include an annular mounting that may resemble the eyepiece of a standard endoscope. This annular mounting is compatible with common optical connectors available from various manufacturers, including Olympus, Karl Storz and others. The second end of the coupler may include a number of embodiments which are intended to allow this end of the coupler to be attached to differing types of endoscopes. For example, in one embodiment, the second end of the coupler includes a cylindrical interior wall for receiving a standard endoscope with an annular eyepiece. Means are provided for securing the endoscope to the second end of the coupler, such as by retaining screws, pins, or other appropriate means. In another form of the first embodiment, the second end of the coupler may include an interior wall having threads which engage with an endoscope having an exterior threaded end instead of the conventional eyepiece. In another form of the first embodiment, the second end of the coupler may include a generally circular recess for receiving an endoscope of the type which has a spring-loaded ball detent. In yet another form of the first embodiment, the second end of the coupler may include an external O-ring for receiving an endoscope of the type having a compression fitting.

In a second preferred embodiment, the second end of the coupler may include a sliding quick release for attachment to the eyepiece of an endoscope. The sliding quick release mechanism is similar to the sliding quick release mechanisms found on many types of optical connectors.

In a third preferred embodiment, the coupler may be comprised of two parts wherein an optical coupler part is prepositioned within the drape and the endoscope coupler part is then attached to the optical coupler part. Having a coupler comprising two parts in some uses makes the coupler easier to use than a coupler having only one integral structure. For the second and third embodiments, in order to effectively seal the drape to the coupler, a locking ring may be used which captures the drape either between the two parts of the two part coupler or between the optical connector and the coupler.

In a fourth preferred embodiment, the coupler may include two parts, namely, an endoscope mount portion and an optical connector mount portion. The fourth embodiment differs from the third embodiment in that the optical connector mount functions similarly to the optical coupler part and locking ring.

The drape may be accordion folded or roll folded so to accommodate the desired packaging configuration prior to use. Conveniently, the drape may also include a pull tab for extending the drape for use. The coupler and drape are generally sterilized by gamma radiation or by gas sterilization and thus may be completely sterile.

According to another novel aspect of this invention, a sterile disposable wand is provided to aide in the attachment of the video camera and optical connector to the coupler. The sterile disposable wand is removably attached to the second end of the coupler prior to connecting the second end with an endoscope. In operation, once the optical connector is attached to the first end of the coupler, the wand can be removed and the endoscope can be coupled to the second end of the coupler.

Stated in another way in broader terms, the invention is a sterile system for coupling a sterile endoscope to an unsterile camera wherein endoscopes are freely interchangeable with the same camera setup so that sterility can be maintained. A coupling means is provided for coupling an unsterile optical connector to a sterile endoscope. The coupling means may be any suitable device having a first end for receiving the unsterile optical connector and a second end for receiving the sterile endoscope. A drape is provided wherein the first end of the coupler remains completely sealed from the sterile environment of the operating room while the second end of the coupler is exposed to the operating room and which allows one to attach the needed endoscope without having to access the optical connector or camera. The sterile drape completely encloses the camera, its trailing cables, and the optical connector. The sterile wand is convenient for stabilizing the video camera and optical connector as they are attached in line to the coupler.

Some video cameras and optical connectors may be partially disinfected by soaking or heating, however, it is quite difficult to completely sterilize these pieces of equipment without incurring tremendous expense due to the cost of labor and equipment associated with surgical sterilization techniques. Thus, a preferred method of endoscopy is one in which unsterile cameras and accessories may be coupled to sterile endoscopes with operating room sterility maintained throughout. With the invention just described, it is possible to use a standard and unsterile surgical video camera and a standard and unsterile optical connector such as a "C" mount connector with a multitude of different types of endoscopes. As described, sterility can be maintained throughout any acts of endoscope manipulation such as changing or adjusting an endoscope with the same camera setup.

Additional advantages of this invention will become apparent from the description which follows, taken in conjunction with the accompanying drawings.

### Brief Description of the Drawings

Figure 1 is a perspective view of the prior art illustrating the eyepiece of a sterile endoscope being directly coupled to an unsterile optical connector wherein the eyepiece is inserted through an opening in the distal end of the drape;
Figure 2 is a perspective view of a sterile surgical coupler and drape constructed in accordance with a first preferred embodiment of this invention;
Figure 3 is a longitudinal cross-sectional view taken along line 3-3 of Figure 2 showing the interior details of the coupler and drape;
Figure 4 is an end view of the first preferred embodiment of the coupler illustrating the coupler having an internal optical pathway therethrough;
Figure 5 is a perspective exploded view of the first preferred embodiment wherein the coupler is detached from the drape and a standard optical connector prior to engagement of the coupler with the optical connector;
Figure 6 is an exploded side view of the first preferred embodiment illustrating the alignment of a camera, optical connector and an endoscope prior to connection;
Figure 7 is a perspective view of the first preferred embodiment illustrating the invention in use wherein an unsterile camera and optical connector are inserted within the drape and connected to the first end of the coupler, and a sterile endoscope is connected to the second end of the coupler;
Figure 8 is a partial fragmentary exploded side view of the first embodiment illustrating an alternative construction of the second end of the coupler which has interior threads for receiving an endoscope of the type having an exterior threaded end;
Figure 9 is an exploded side view of the first embodiment illustrating another alternative construction of the second end of the coupler which includes a generally circular recess for receiving an endoscope of the type having a spring-loaded ball detent connection;
Figure 10 is an exploded side view of the first embodiment illustrating yet another alternative construction of the second end of the coupler which includes an external O-ring for receiving an endoscope of the type having a compression fitting connection;
Figure 11 is a perspective exploded view of a second preferred embodiment of the coupler and drape of this invention illustrating the coupler detached from the drape and optical connector and also detached from the standard endoscope prior to use;
Figure 12 is an exploded side view of the second preferred embodiment, as shown in Figure 11, illustrating the alignment of a video camera, optical connector and an endoscope prior to connection;
Figure 12a is an enlarged fragmentary partial cross-sectional view of the locking ring in Figure 12, illustrating the use of a spring washer;
Figure 12b is an enlarged fragmentary partial cross-sectional view of the locking ring in Figure 12, illustrating the use of a Teflon® seal in lieu of a spring washer;
Figure 13 is a longitudinal cross-sectional view, showing the interior details of the second embodiment of the coupler and drape of this invention when in use with a sterile camera setup including an optical connector and endoscope;
Figure 14 is a perspective exploded view of a third preferred embodiment of the coupler and drape of this invention illustrating the coupler detached from the drape and optical connector and also detached from a standard endoscope, prior to use;
Figure 15 is an exploded longitudinal cross-sectional side view of the second preferred embodiment of the coupler and drape of this invention illustrating the alignment of a video camera, optical connector, and an endoscope prior to connection;
Figure 16 is a perspective exploded view of a fourth preferred embodiment of the coupler and drape of this invention, wherein the coupler is detached from the drape and optical connector and also detached from a standard endoscope, prior to use;
Figure 17 is an exploded longitudinal cross-sectional side view of the fourth preferred embodiment of the coupler and drape of this invention illustrating the alignment of a video camera, optical connector and endoscope prior to connection;
Figure 18 is a perspective view of the fourth preferred embodiment of the coupler and drape of this invention illustrating the invention as it would be packed prior to use, wherein a sterile disposable wand is connected to the second end of the coupler and the drape is extended over the second end of the coupler; and
Figure 19 is another perspective view of the fourth preferred embodiment, as shown in Figure 18, illustrating the drape being pulled back over the first end of the coupler and wherein the sterile disposable wand is separated from the second end of the coupler so that the endoscope may then be connected thereto.

### Best Mode for Carrying out the Invention

Typically, a sterile endoscope is connected by means of an optical connector to a surgical camera. The distal end of the endoscope can be introduced into an internal body sight for viewing. The maintenance of sterility in these types of medical procedures is critical. While there does exist sterile cameras and optical connectors, it is more common in the art to encounter surgical cameras and optical connectors that are unsterile and that are shielded from the sterile environment of the operating room by means of a sterile drape or bag.

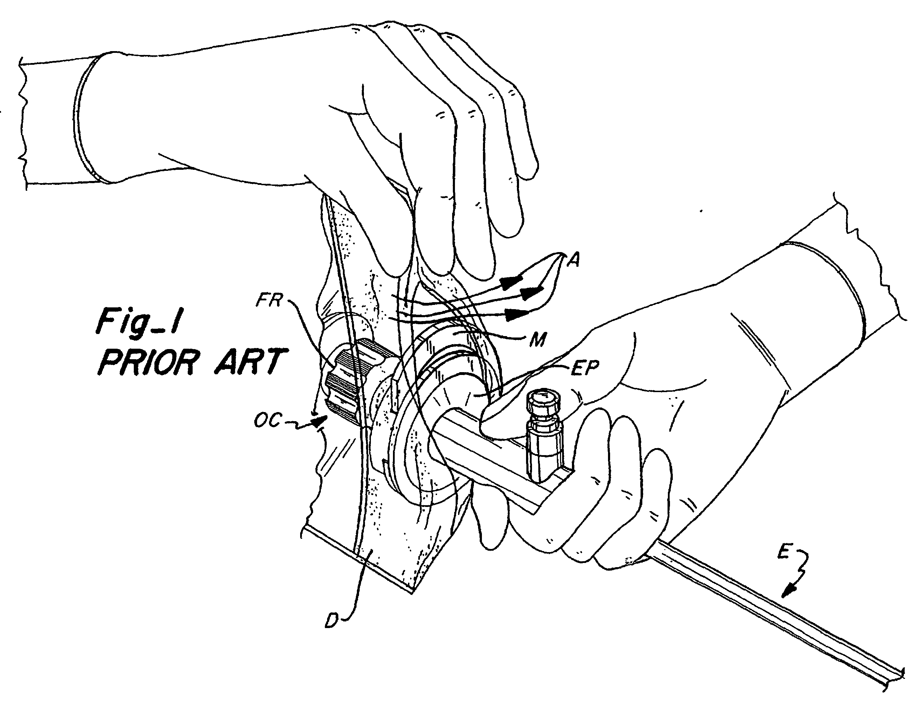

The invention disclosed herein is advantageous over the prior art because sterility can be maintained during endoscopic procedures. In the prior art method and device of Figure 1, a standard endoscope E with eyepiece EP is inserted within drape D. The act of inserting the eyepiece EP into the drape D and coupling the eyepiece EP to an unsterile mount portion M of optical connector OC allows contamination to travel from the inside of the drape D to the sterile environment of the operating room. This path of contamination is generally shown by arrows A. Further contamination can occur by manipulation of the endoscope to achieve correct camera alignment and by sealing the open distal end of the drape to the protruding distal portion of the endoscope. Assuming the interior of the drape is sterile prior to the insertion of a camera and optical connector, the invention disclosed herein is still advantageous over the prior art. That is, sterility in the prior art can be maintained by first inserting the endoscope through the opening in the distal end of the drape and then sealing the drape with respect to the endoscope prior to introducing the unsterile camera and optical connector into the drape. However, if it were desired then to change the type of endoscope used with the existing camera setup, the sterile barrier between the camera and the distal end of the endoscope would have to be broken by reopening the sealed end of the drape. This act can result in contamination and is therefore an undesirable method of interchanging endoscopes with the same camera setup. As will be discussed, the invention disclosed herein allows endoscopes to be freely interchanged and ensures the maintenance of sterility during endoscope procedures.

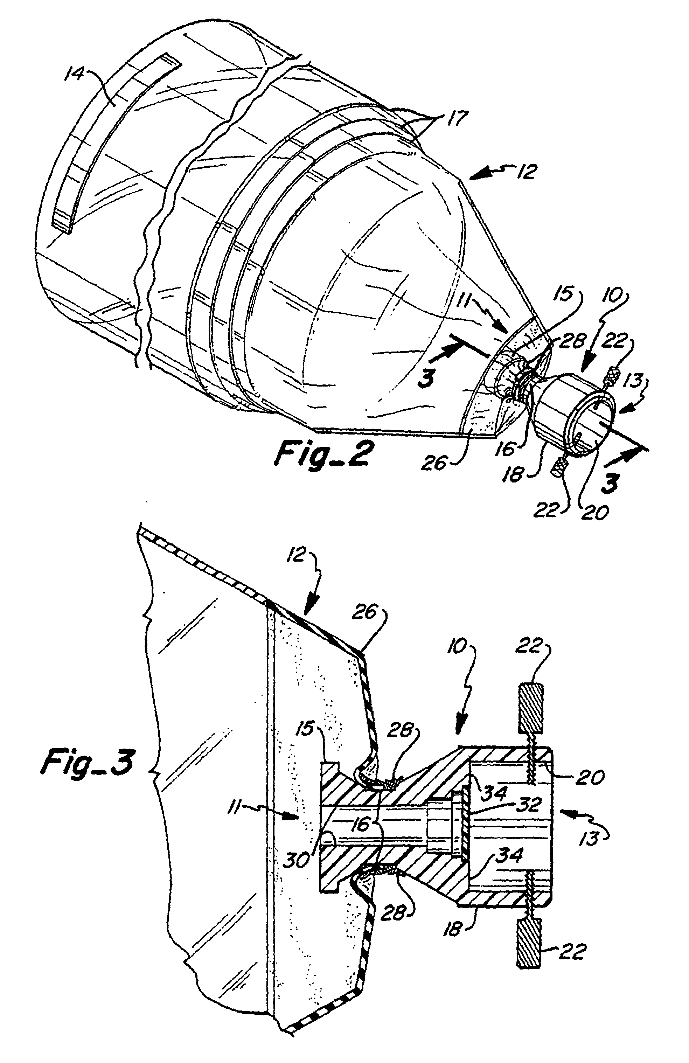

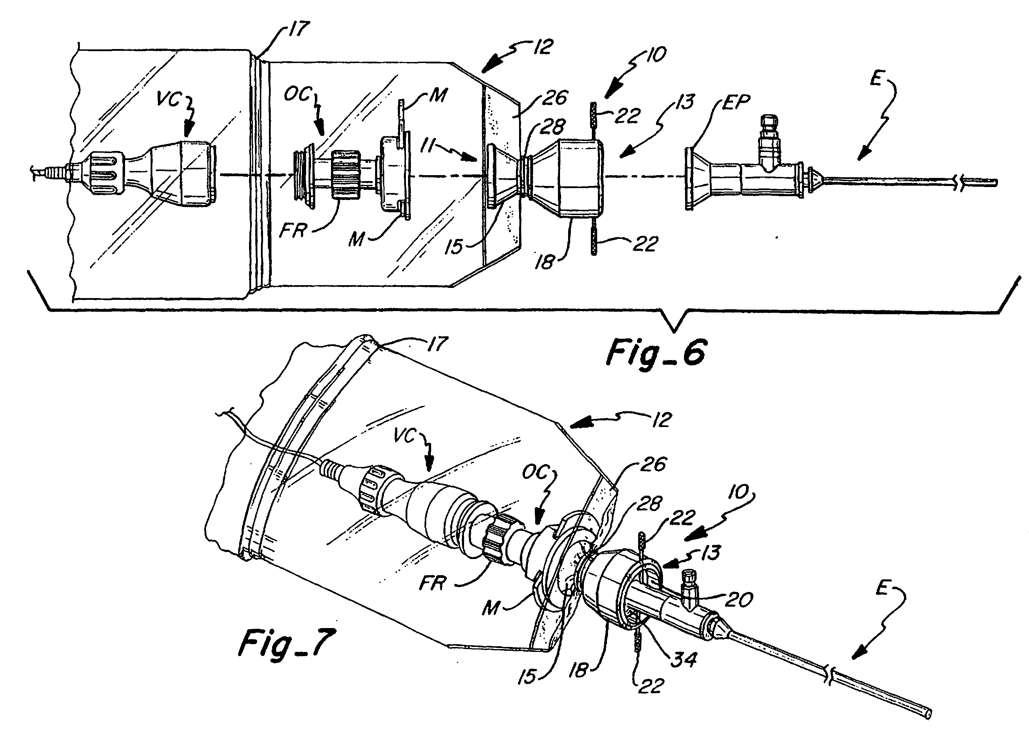

In accordance with this invention, in a first preferred embodiment, a disposable sterile coupler 10 and attached sterile drape 12, as best seen in Figures 2 and 6, is provided for coupling an unsterile camera setup, comprising an unsterile video camera VC and optical connector OC having a focusing ring FR, to a sterile endoscope E. The overall structure of the sterile coupler 10 and drape 12 can best be seen by viewing Figures 2 and 3. The sterile coupler 10 includes a first end 11 having an annular mounting 15 which attaches to a common optical connector such as a "C" mount connector. This annular mounting 15 resembles the eyepiece of a standard endoscope. The coupler 10 further includes a second end 13 having an endoscope mount 18 characterized by a substantially cylindrical shape which may be configured to match the particular type of endoscope used. As best seen in Figures 3 and 6, in the first preferred embodiment, the endoscope mount 18 includes an interior cylindrical wall 20 for receiving a standard endoscope E having an annular eyepiece EP. Between the annular mounting 15 and endoscope mount 18 is a neck portion 16. An annular mounting 15 is inserted within an opening located at the distal end 26 of the drape 12 such that the opening surrounds neck portion 16. A sealing means 28 such as surgical tape may be used to seal the distal end 26 against the neck portion 16, thus providing a sterile barrier between the first and second ends of the coupler 10. Sealing and bonding of the drape 12 to the coupler 10 may also be done by a variety of methods, including adhesives, shrink-wrap or double-faced adhesive strips.

Sterile drape 12 may include folds 17 in order to reduce the size of the drape for storage prior to use. As shown in Figure 2, folds 17 may be telescopic wherein consecutive drape sections are folded on top of one another, or alternatively folds 17 may be folded in a roll configuration (not shown) like a condom. If folds 17 are telescopic, pull tab 14 may be provided in order to extend the drape for use. As seen in Figure 3, the primary purpose of coupler 10 is to provide an optical pathway and sterile barrier between a sterile endoscope E and an unsterile camera setup including video camera VC and optical connector OC. Accordingly, interior passageway 30 is provided to allow light to be transmitted from the endoscope E to the video camera VC. To maintain sterility, optically clear window 32 is provided which allows the passage of light and provides a sterile barrier between the video camera VC and endoscope E. As best seen in Figures 3 and 6, for use of the coupler 10 with an endoscope E of the type having a conventional eyepiece EP, the eyepiece EP is inserted within endoscope mount 18 such that the eyepiece EP is pressed flush against interior wall 34 and window 32. Retaining screws 22 may then be used to secure the eyepiece EP. Alternatively, endoscope mount 18 could be configured like mount portion M of optical connector OC in order to receive the standard eyepiece EP of an endoscope. Other methods of securing the mount 18 to the endoscope are possible within the intended scope of this disclosure as will be discussed below.

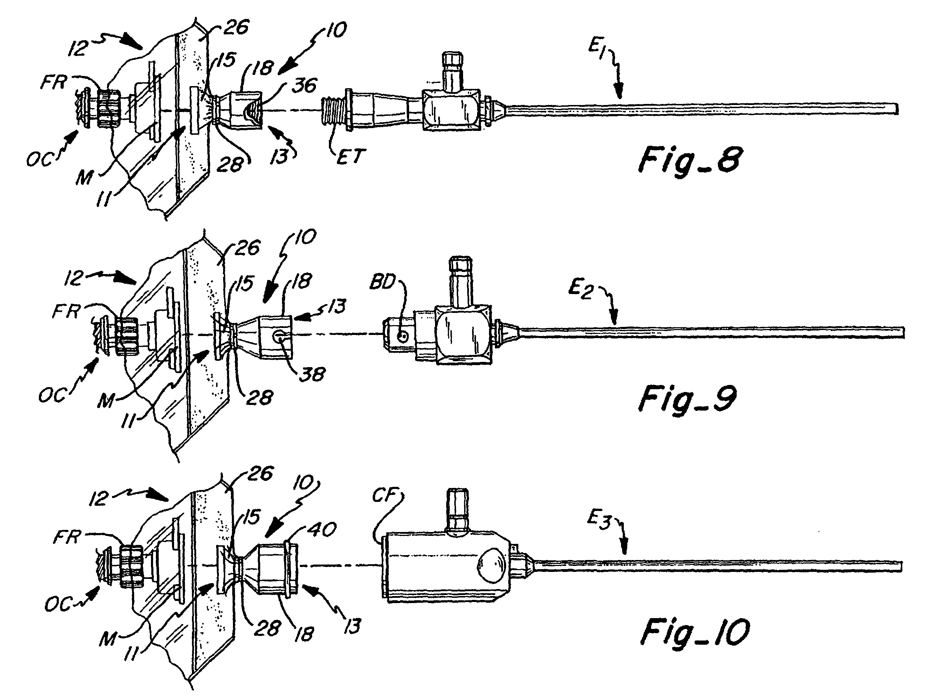

In addition to the construction of endoscope mount 18 as illustrated in Figures 2 through 7 of the first embodiment, endoscope mount 18 may be configured to match any number of differing types of endoscopes. For example, as shown in Figure 8, endoscope mount 18 may include interior threads 36 for which to receive exterior threads ET of endoscope E₁. As shown in Figure 9, endoscope mount 18 may include a generally circular recess 38 for which to receive ball detent BD of endoscope E₂. In yet another arrangement, as shown in Figure 10, endoscope mount 18 may include an O-ring 40 for which to receive an endoscope E₃ that has a compression fitting CF.

The coupler 10 can be made of a suitable plastic or metal material which is sterilizable by various methods such as gas sterilization or gamma radiation and thus is made completely sterile. A suitable material for the coupler may be polycarbonite or PETG, or possibly acrylic or styrene. Similarly, the sterile drape 12 may be made out of a material such as polyethylene preferably from 1 to 6 mils in thickness, that is sterilizable also making the drape completely sterile.

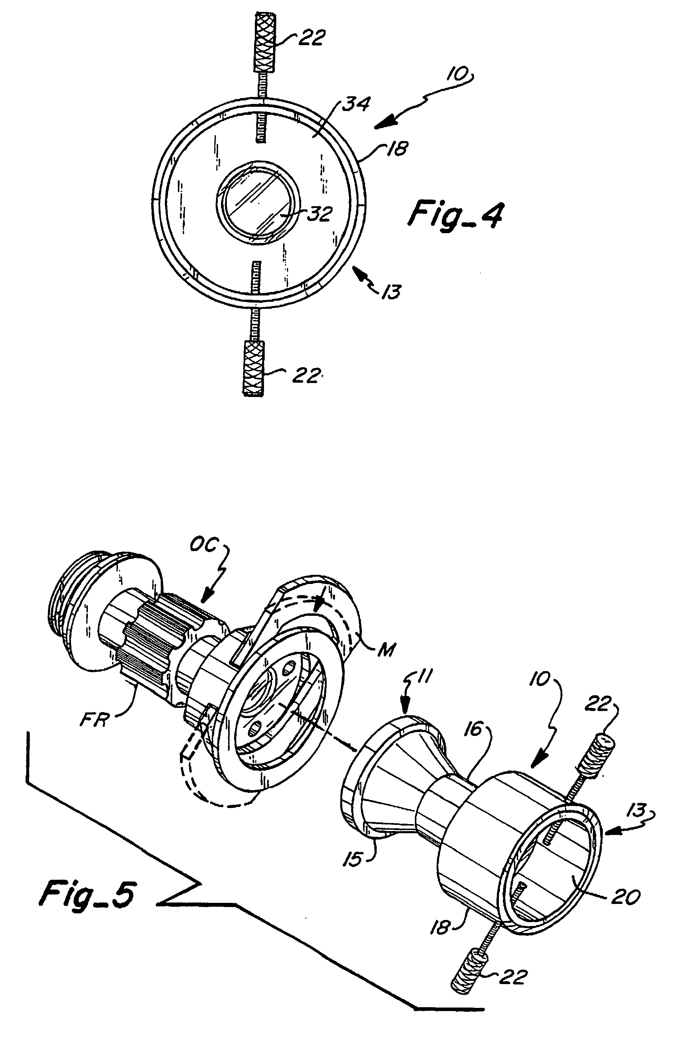

In use of the first embodiment, as best seen in Figures 4-6, video camera VC and optical connector OC are inserted within the proximal end of sterile drape 12. Mounting portion M of optical connector OC is coupled to annular mounting 15 of coupler 10. Video camera VC may then be attached to connector OC. Sterile drape 12 is then pulled back over the optical connector OC, video camera VC and its trailing cables thus providing a sterile covering isolating the unsterile camera setup from the sterile operating environment. Sterile endoscope E may then be coupled with endoscope mount 18 of coupler 10. The endoscope E may be secured by means of retaining screws 22 or other appropriate securing means. If it is desired to use a different type of endoscope having differing optical qualities, retaining screws 22 are simply released and endoscope E is removed from mount 18. A new endoscope may then be introduced wherein sterility is maintained during the change in endoscopes. After use, the optical connector OC and endoscope E are disconnected from the coupler 10, and the drape 12 and coupler 10 are thrown away.

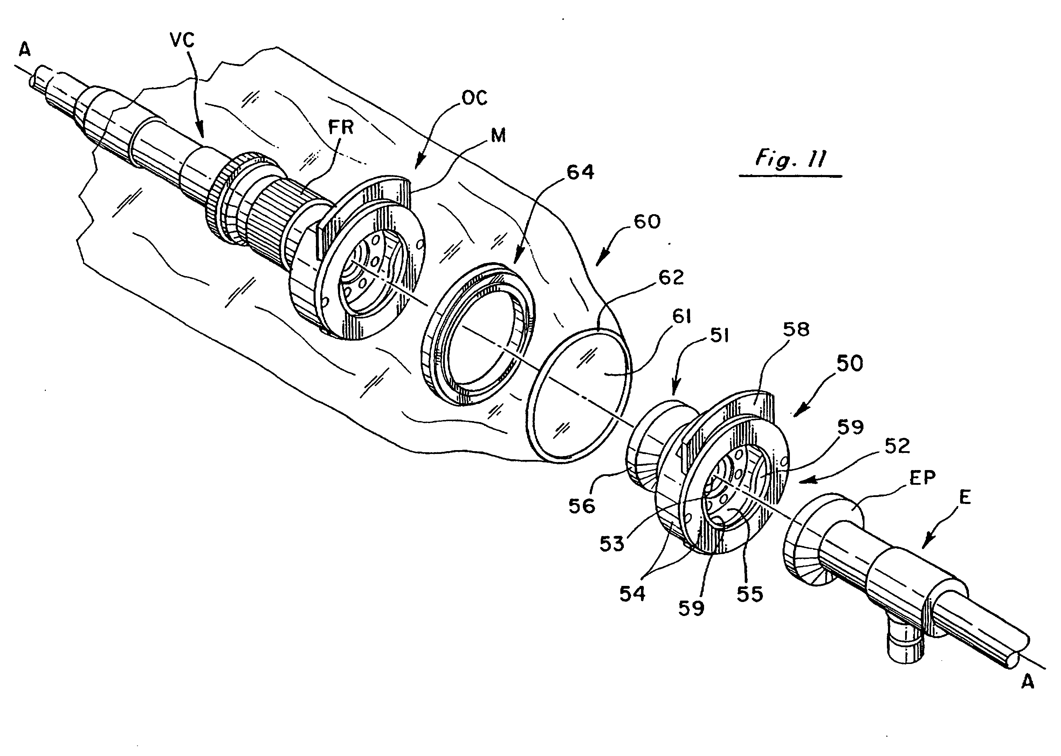

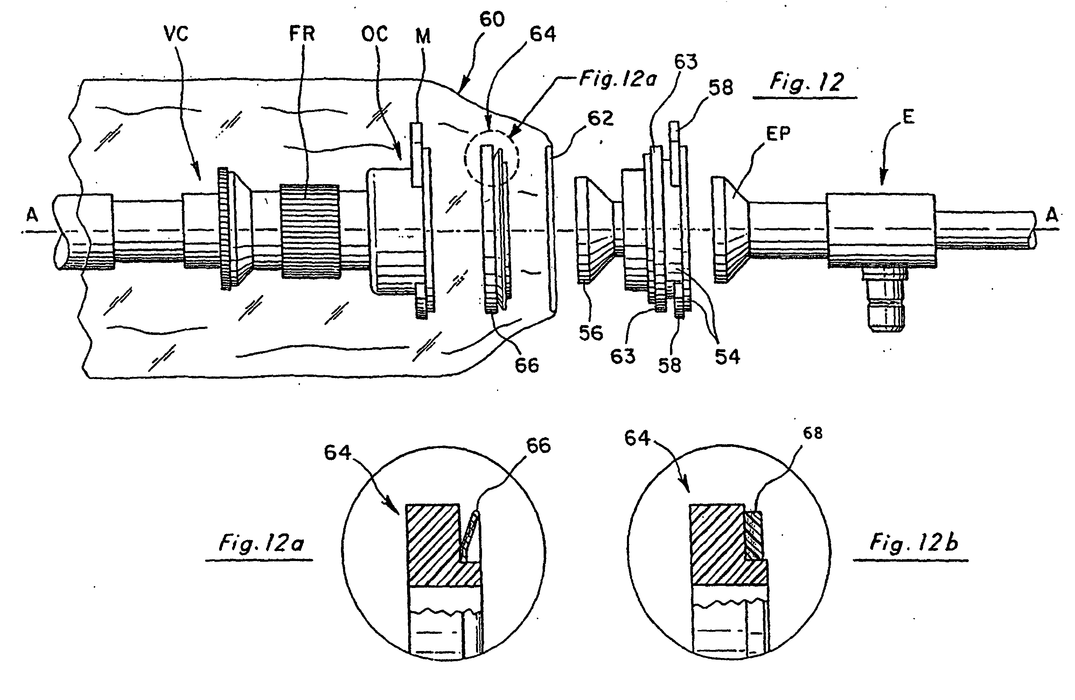

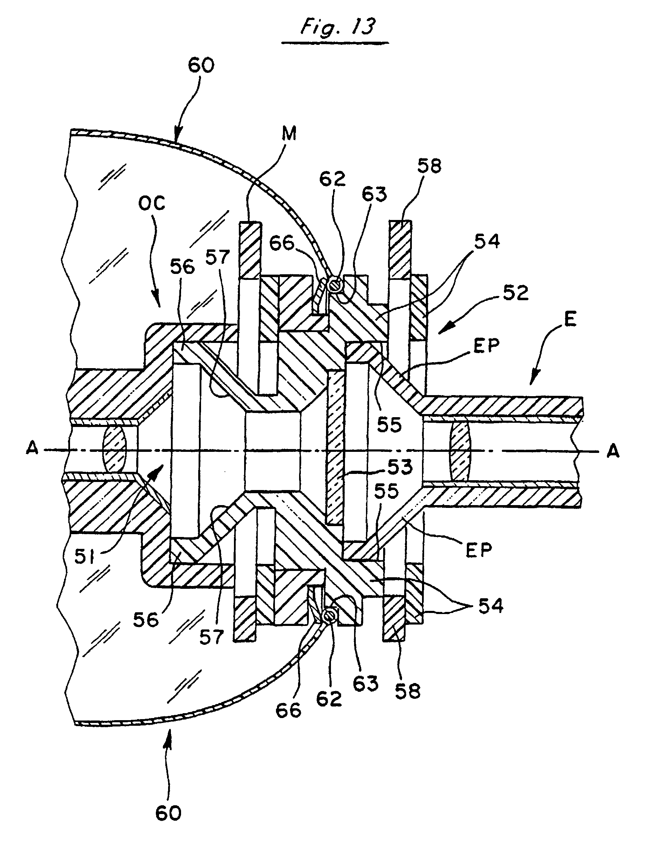

In a second preferred embodiment of the sterile surgical coupler and drape of this invention, a sterile coupler 50 is provided, as best seen in Figures 11-13. The sterile coupler 50 includes a first end 51 which attaches to the optical connector OC, and a second end 52 which connects to the endoscope E. As with the first embodiment, a window 53 is positioned within the coupler 50 for providing a sterile barrier between the optical connector OC and the sterile endoscope E. Coupler 50 includes an endoscope mount 54 having an interior passageway 55 which optically communicates with the window 53. Furthermore, the coupler 50 includes an optical coupler mount 56 also including an interior passageway 57 which communicates with the opposite side of window 53. In order to attach the coupler 50 to a desired endoscope E, coupler 50 includes a securing means such as sliding quick release mechanism 58 which is similar to the mount portion M of optical connector OC. Mechanism 58 is mounted transversely with respect to axis A-A which defines the longitudinal direction. In operation, sliding quick release 58 may be depressed to enlarge the opening within interior passageway 57 enabling an endoscope to be inserted therein. Upon relieving pressure on the sliding quick release 58, V portion 59 of quick release 58 then engages the eyepiece EP of the endoscope E. A spring member (not shown) disposed within endoscope mount 54 provides the sliding quick release 58 with spring tension to releasably engage the endoscope E. According to the second embodiment, drape 60 includes a drape ring 62 which may be constructed of a circular shaped wire that is integral with the distal end of the drape. Drape ring 62 defines the drape opening 61. Figure 11 illustrates the arrangement of the sterile coupler 50 with respect to optical connector OC and locking ring 64 when the drape and coupler of this invention is in use. However, prior to use, the sterile coupler and drape of this invention is packaged such that the sterile drape extends over the second end 52 of the coupler 50 with the locking ring 64 exposed exteriorly of the drape 60. Thus, it will be understood that when in use, the drape 60 is inverted so that it is pulled back over the first end 51 of the coupler 50 and completely over optical connector OC, video camera VC and its trailing cables. As best seen in Figures 12a and 13, locking ring 64 may include sealing means such as spring washer 66 to help provide a water and airtight seal for capturing the drape 60 therebetween. More specifically, endoscope mount 54 includes an engagement flange 63 which receives drape ring 62.
The contact of washer 66 against drape ring 62 which is pressed against flange 63 ensures a tight seal. In lieu of spring washer 66, as shown in Figure 12b, a Teflon® seal 68 may be used to provide the liquid and airtight seal.

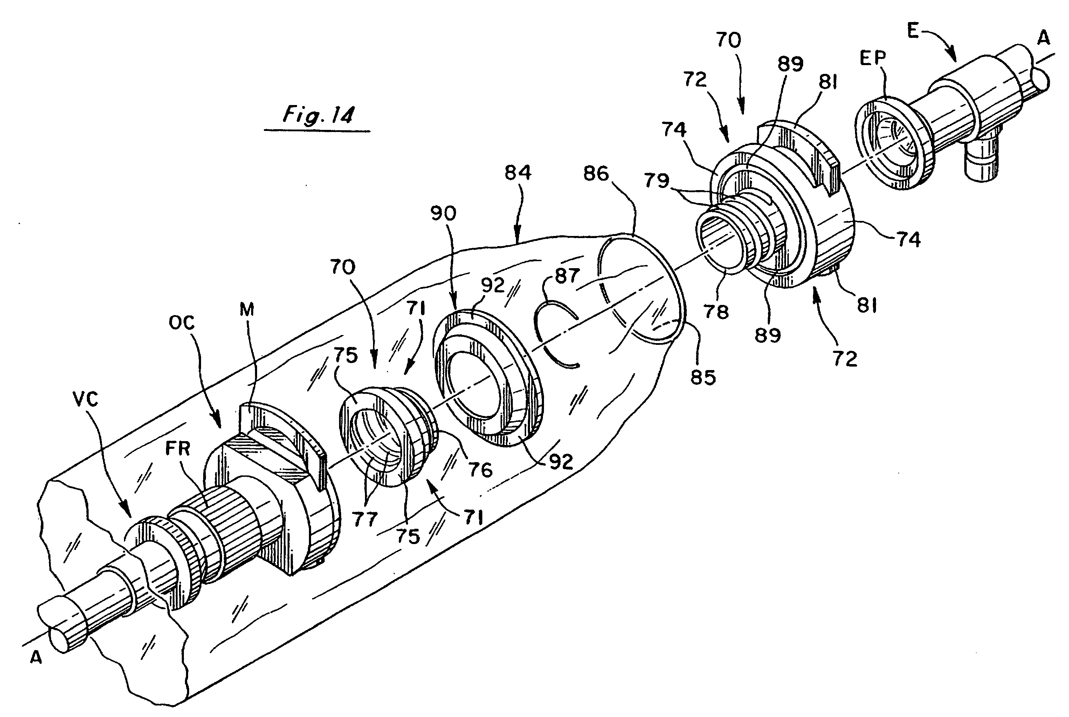

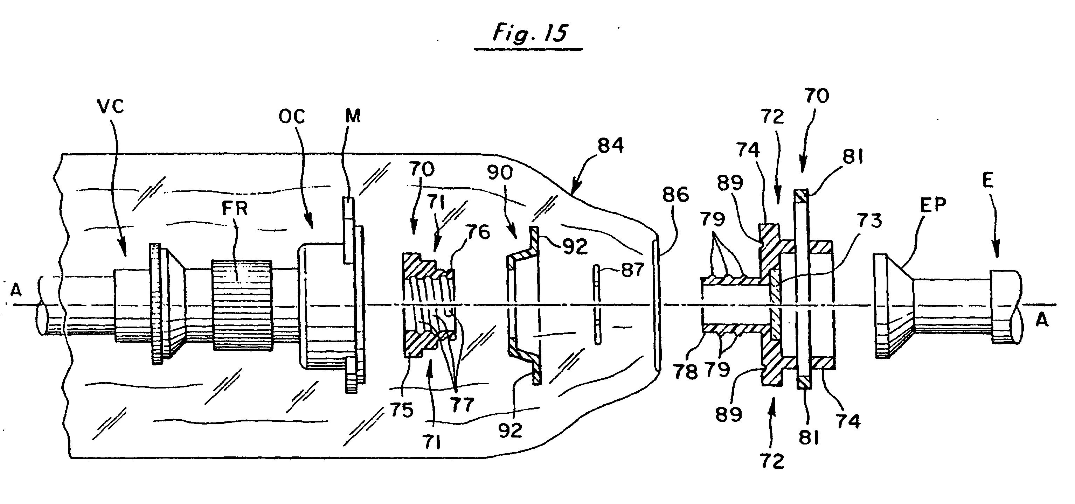

In a third preferred embodiment of the coupler and drape of this invention, a sterile coupler 70 is provided which comprises two main structural elements, namely, an optical coupler part 71 and an endoscope coupler part 72. This third embodiment is best seen in Figures 14 and 15. As with the second embodiment, the positioning of the drape is illustrated as when the drape and coupler are in use in surgery. That is, when packaged, the drape is initially extended over endoscope coupler part 72 but then is reversed and pulled back over optical coupler part 71. Endoscope coupler part 72 includes a window 73 to provide a sterile barrier between the unsterile optical connector OC and video camera VC and the sterile endoscope E. Coupler part 72 further includes endoscope mount 74 and sliding quick release 81 which serves as the structure for attachment to endoscope E. Flange portion 78 of endoscope coupler part 72 includes a plurality of externally exposed threads 79 which mate with internal thread 77 of optical coupler part 71. Optical coupler part 71 includes optical coupler mount 75 which is engaged by the mount portion M of optical connector OC. Internal threaded portion 77 of optical coupler part 71 mates with external threads 79 to provide a positive locking connection. Flange 76 of optical coupler part 71 is insertable through locking ring 90, locking wire 87 and ultimately slides over flange 78 so that threads 79 may engage internal threaded portion 77. Locking ring 90 includes a flange 92 which assists in capturing the drape 84 between ring 90 and coupler part 72. As with the second embodiment, drape 84 includes a drape ring 86 defining a drape opening 85. In order provide a watertight and airtight seal, drape ring 86 engages with groove 89 formed on endoscope mount 74. Locking wire 87 serves to stablize the connection between optical coupler part 71 and locking ring 90.

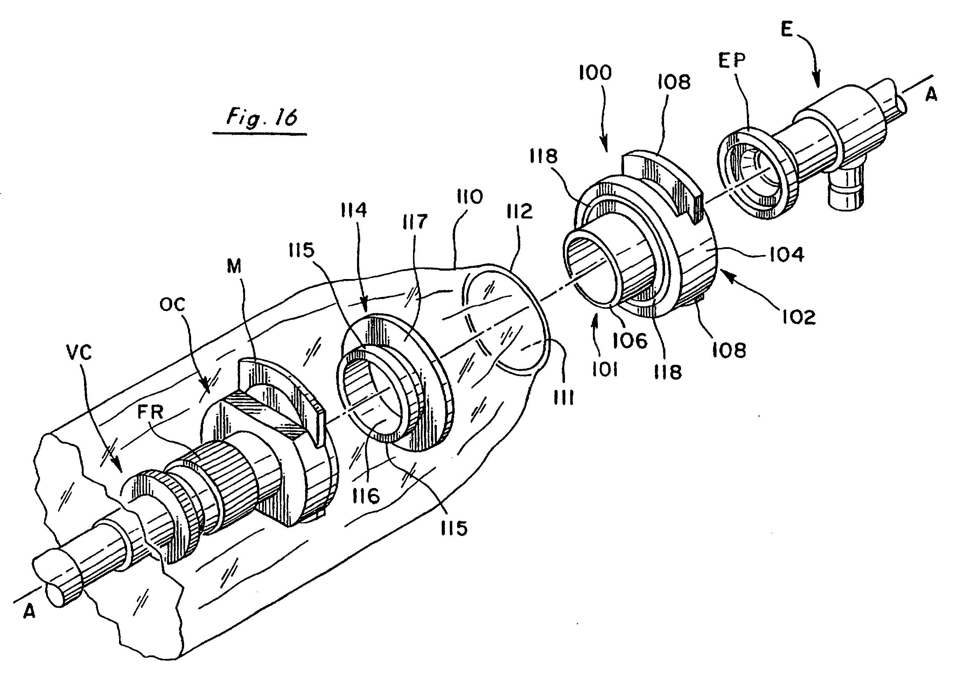

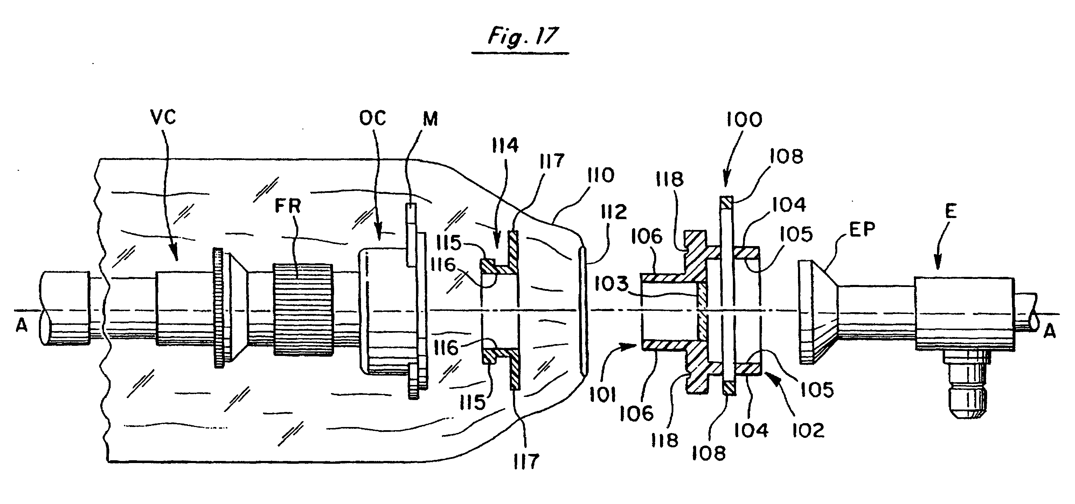

According to a fourth preferred embodiment of the coupler and drape of this invention, as best seen in Figures 16 and 17, a sterile coupler 100 is provided. The fourth embodiment is similar to the second embodiment, however in the fourth embodiment, structure corresponding to the optical coupler mount 56 of the second embodiment is made integral with the locking ring 64, as will be explained below. Sterile coupler 100 includes a first end 101 which is engageable with the optical connector OC. Second end 102 is engageable with an endoscope E. A window 103 provides a sterile barrier between the optical connector OC and the endoscope E. Coupler 100 further includes an endoscope mount 104 which attaches to the endoscope E by means of sliding quick release 108. The endoscope E is received within interior passageway 105 wherein V portion (not shown) engages the eyepiece EP of the endoscope E. Mount 104 includes annular flange 106 which has a smooth exterior surface. Drape 110 includes a drape ring 112 defining a drape opening 111. Locking ring 114, functionally corresponding to locking ring 64 of the second embodiment, also includes an optical coupler mount 115 and interior passageway 116 which functionally correspond to optical coupler mount 56 of coupler 50. In use, the drape 110 extends over the first end 101 of coupler 100 and locking ring 114 is engaged with annular flange 106 of endoscope mount 104. A close tolerance fit between flange 106 and interior passageway 116 of locking ring 114 provides a positive locking connection. Optical connector OC engages optical coupler mount 115 in the same manner the optical connector OC engages optical coupler mount 56. Flared flange 117 of locking ring 114 provides an enlarged surface area in which to grasp and hold drape 110 between endoscope mount 104 and locking ring 114. Endoscope mount groove 118 provides a matching seat for drape ring 112.

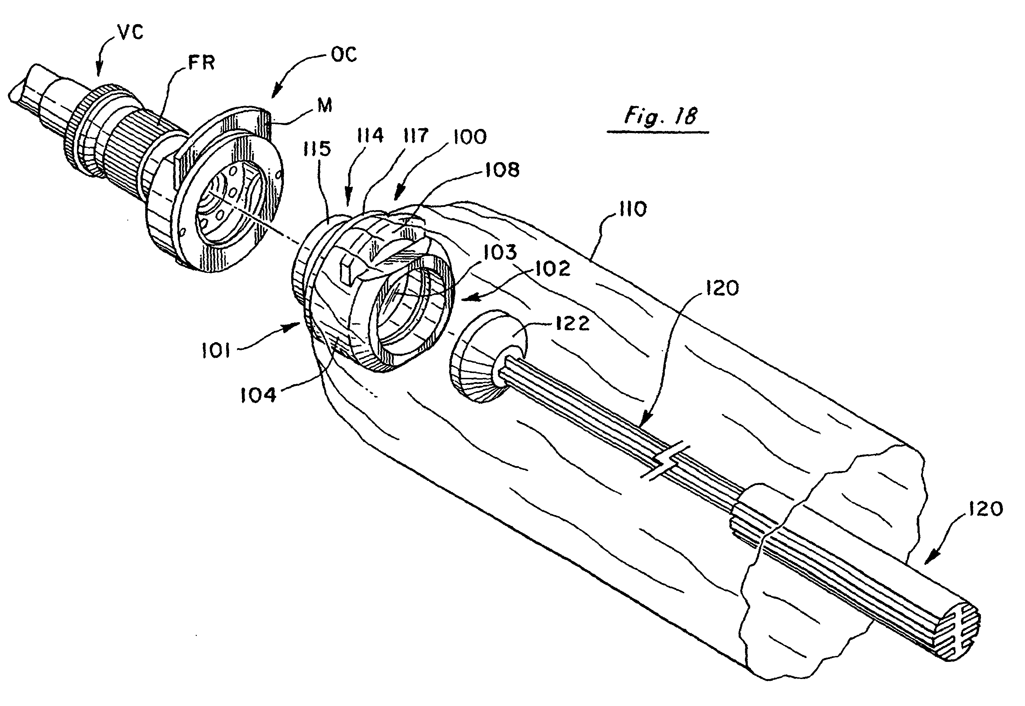

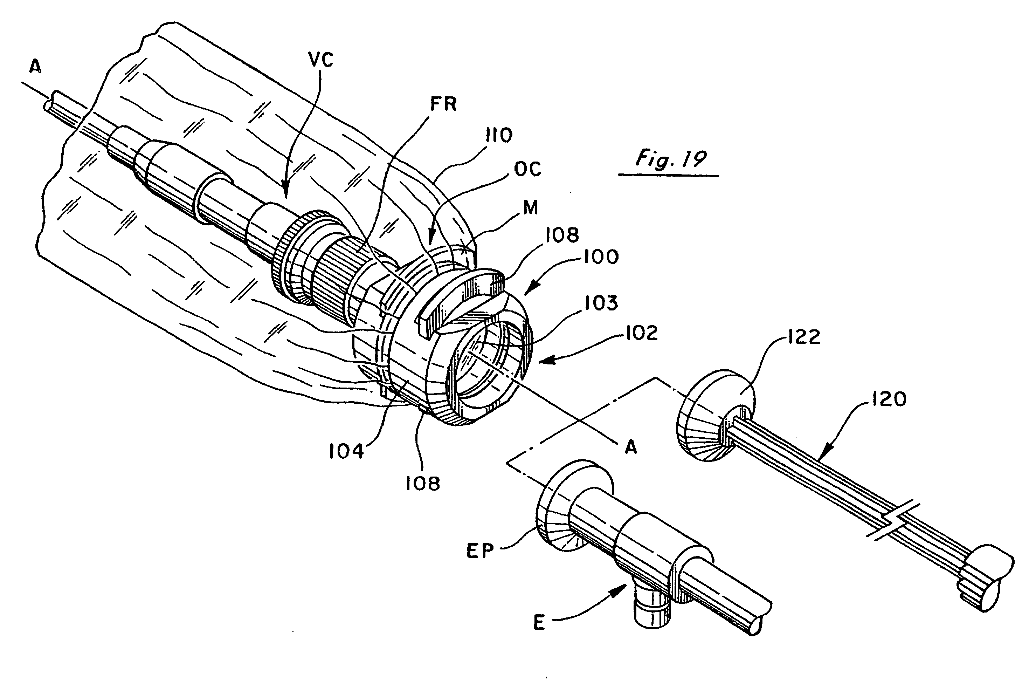

According to another novel aspect of this invention, a sterile disposable wand or stick 120 may initially be engaged with sterile couplers 10, 50, 70 or 100 to help stablize the sterile coupler and drape when the unsterile camera setup is connected to the sterile coupler. The sterile disposable wand 120 is shown in Figures 18 and 19. After engaging the unsterile camera setup with the coupler, the drape is pulled back over the camera, optical connector and trailing cables. The disposable wand 120 is then disengaged from the second end of the sterile coupler. As illustrated, the distal end of wand 120 includes an eyepiece portion 122 resembling the eyepiece of an endoscope. This configuration of eyepiece portion 122 enables the wand 120 to easily be engaged by the sliding quick releases 58, 81 and 108 of the second, third and fourth embodiments, respectfully. Alternatively, the distal end of wand 120 may simply be of a cylindrical shape to engage with the cylindrical interior wall 20 of the first embodiment.

When packaged prior to use, the sterile disposable wand 120 is pre-inserted into the second end of the coupler so that upon opening the packaged contents, the wand may be grasped by a sterile nurse who assists in stablizing the connection of the unsterile camera setup to the first end of the coupler.

Although the preferred embodiments disclosed herein illustrate the optical connector OC disposed within the drape, it is also within the spirit and scope of this invention to provide a coupler which is attachable to an optical connector positioned exteriorly of the drape. That is, some optical connectors are available which are autoclavable and thus can be made completely sterile. Thus, a particular surgeon may desire to focus an image onto the video camera by manipulating the focus ring exteriorly of the drape. For example, for the second embodiment shown in Figure 11, the optical connector OC with focusing ring FR could be directly attached to the coupler 50, and a modified locking ring (not shown) could be used to connect the video camera VC to the optical connector OC which is placed exteriorly of the drape 60. Thus, an airtight and fluid-proof seal would be created between the video camera VC and the optical connector OC.

With this apparatus and method, a standard surgical camera and optical connector can be used which does not need to be sterilized through heating, soaking, or other sterilizing procedures and in which a number of endoscopes may be used with the same camera setup. The advantages of sterility and interchangeability of endoscopes makes this invention attractive to medical clinics and hospitals who often times cannot afford other endoscopic systems which, in order to maintain sterility, are much more complex and more difficult to use.

As discussed above in the prior art, an endoscope is typically attached to the optical connector which meant that in order for an endoscope to be placed in use, the sterile eyepiece of the endoscope had to be inserted within the drape and then directly attached to the unsterile optical connector. During this process, contamination is allowed to freely travel from the inside of the drape where the unsterile camera and optical connector are positioned, to the outside sterile environment of the operating room by means of the hole located in the distal end of the drape. Thus, according to the device and method of the invention disclosed herein, by isolating the first end of the coupler within the drape when the drape is extended for use over the unsterile camera, optical connector, and trailing cables, sterility may be maintained when subsequently attaching the endoscope to the coupler and for subsequent acts of changing endoscopes with the same camera setup.

## Claims

1. An apparatus for optically coupling a sterile endoscope (E) to a sterile camera setup including an unsterile optical connector (OC) mounted to an unsterile camera (UC) having trailing cables, and for enclosing the unsterile camera setup in a sterile enclosure for use of the unsterile camera setup in a sterile surgical environment, said apparatus comprising, a sterile coupler (50, 70, 100) having a first end including a first mounting structure (56, 71, 115) for connection to the unsterile optical connector (OC), and having a second end including a second mounting structure (54, 72, 104) for connection to the sterile endoscope (E), said sterile coupler further having an optical path positioned between said first and second ends thereof through which an image may be transmitted from the sterile endoscope to the unsterile camera, a sterile barrier (53, 73, 103) positionable between said first and second ends of said sterile coupler,
a sterile drape (60, 84, 110) having an open distal end and including a drape ring (62, 86, 112) formed thereon, said sterile drape positionable over said first end of said coupler and having a proximal end extendable over the unsterile optical connector, the unsterile camera and its trailing cables; and means for sealing said distal end of said drape between said first and second ends of said sterile coupler whereby said first end of said coupler is isolated from the sterile surgical environment located outside said drape, **characterized in that** said sealing means includes
a locking ring (64, 90, 114) positionable between said first end and said second end of said sterile coupler, and engaging said drape ring to compress the drape ring against the coupler between said locking ring and said coupler.

2. An apparatus, as claimed in claim 1, wherein said sterile barrier includes:
an optically clear window (53, 73, 103) mounted transversely in said optical path.

3. An apparatus, as claimed in claim 1, wherein said second mounting structure includes:
an interior passageway (55) for receiving the sterile endoscope; and
securing means (58) attached to said second mounting structure for releasably attaching said second mounting structure to the sterile endoscope.

4. An apparatus, as claimed in claim 3, wherein said securing means (58) includes a sliding quick release.

5. An apparatus, as claimed in claim 1, wherein said second mounting structure includes:
a transversely mounted securing means (58, 81, 108) for releasably attaching said second mounting structure to the sterile endoscope.

6. An apparatus, as claimed in claim 1, wherein said sterile coupler further includes:
an integral engagement flange (63); and
said drape ring (62) being attached at said distal end of said drape for releasable engagement with said engagement flange (63) and said locking ring (64).

7. An apparatus, as claimed in claim 1, wherein said locking ring further includes:
a sealing member (66, 68) attached to one side of said locking ring for capturing the drape ring (62), the sealing member providing an airtight and liquid proof seal between said optical connector and said sterile coupler.

8. An apparatus, as claimed in claim 1, wherein said locking ring further includes:
a flange (92,117) for capturing the drape ring (86,112) in a groove (89,118) formed on the sterile coupler (70,100), the flange providing an airtight and liquid proof seal between said optical connector and said sterile coupler.

9. An apparatus, as claimed in claim 1, further including:
a sterile disposable wand (120) attachable to said second end of said sterile coupler for providing support to the camera setup when being attached to said sterile coupler.

10. An apparatus, as claimed in claim 1, wherein:
said first mounting structure includes an optical coupler part (71) having an internal threaded portion (77), and said second mounting structure includes an endoscope coupler part (72) having external threads (79) for engaging said internal threaded portion (77) to releasably secure said distal end of said drape therebetween.

11. An apparatus, as claimed in claim 10, wherein:
said locking ring further includes a flange (92) which captures the distal end of said drape against an endoscope mount (74) of said endoscope coupler part (72).

12. An apparatus, as claimed in claim 1, wherein:
said first mounting structure includes an optical coupler mounting (115) and an interior passageway (116) formed through said optical coupler mounting; and
said second mounting structure includes an endoscope mounting (104) having an annular flange (106) for slidably engaging with said interior passageway (116) to releasably secure said distal end of said drape between said optical coupler mounting (115) and said endoscope mounting (104).

13. An apparatus, as claimed in claim 12, wherein:
said optical coupler mounting (115) is attached to said locking ring (114).

14. A method of coupling an unsterile camera setup including an optical connector (OC) and camera (C) to a sterile endoscope (E) wherein differing endoscopes may be freely interchanged without compromising the sterility of a surgical environment, said method comprising the steps of providing a sterile coupler (50, 70, 100) having a first end attachable to the unsterile camera setup and a second end attachable to the sterile endoscope, providing a sterile drape (60, 84, 110) having an open distal end including a drape ring (62, 86, 112) formed thereon positionable over the first end of the coupler and having a proximal end extendable over the camera setup inserting the unsterile optical connector (OC) mounted to the unsterile camera (C) within the proximal end of the drape (60, 84, 110), attaching the unsterile optical connector the first end of the coupler, and attaching the sterile endoscope to the second end of the coupler **characterized in that** said method further comprises the step of:
sealing the distal end of the drape (60, 84, 110) around the first end of the coupler (50, 70, 100) by a locking ring (64, 90, 114) engageable with the drape ring and said coupler thereby providing a sterile seal wherein the first end of the coupler is sealed within the drape and the second end of the coupler is exposed to the sterile surgical environment.

15. A method, as claimed in claim 14, further including the steps of:
supporting the sterile coupler with a sterile disposable wand (120) attached to the second end thereof prior to attaching the unsterile optical connector (OC) to the first end of the coupler; and
detaching the sterile disposable wand (120) from the second end of the sterile coupler prior to attaching the sterile endoscope to the second end of the coupler.

## Patentansprüche

1. , Vorrichtung zum optischen Verbinden eines sterilen Endoskops (E) mit einer sterilen Kameraanordnung, die einen nicht sterilen optischen Verbinder (OC) enthält, der an einer nicht sterilen Kamera (UC) angebracht ist und Stromzuführungskabel aufweist, und zum Einschließen der nicht sterilen Kameraanordnung in einer sterilen Umhüllung zum Einsatz der nicht sterilen Kameraanordnung in einer sterilen chirurgischen Umgebung, wobei die Vorrichtung eine sterile Kupplung (50, 70, 100) umfasst, die ein erstes Ende aufweist, das eine erste Anbringungsstruktur (56, 71, 115) zur Verbindung mit dem nicht sterilen optischen Verbinder (OC) enthält, und ein zweites Ende, das eine zweite Anbringungsstruktur (54, 72, 104) zur Verbindung mit dem sterilen Endoskop (E) enthält, wobei die sterile Kupplung des Weiteren einen Lichtweg aufweist, der zwischen dem ersten und dem zweiten Ende derselben angeordnet ist und über den ein Bild von dem sterilen Endoskop zu der nicht sterilen Kamera übertragen werden kann, und eine sterile Sperre (53, 73, 103), die zwischen dem ersten und dem zweiten Ende der sterilen Kupplung angeordnet werden kann;
einen sterilen Überzug (60, 84, 110), der ein offenes vorderes Ende aufweist und einen daran ausgebildeten Überzugsring (62, 86, 112) enthält, wobei der sterile 0-berzug über dem ersten Ende der Kupplung angeordnet werden kann und ein hinteres Ende aufweist, das über den nicht sterilen optischen Verbinder, die nicht sterile Kamera und ihre Stromzuführungskabel ausgedehnt werden kann, sowie eine Einrichtung zum Abdichten des vorderen Endes des Überzugs zwischen dem ersten und dem zweiten Ende der sterilen Kupplung aufweist, so dass das erste Ende der Kupplung gegenüber der sterilen chirurgischen Umgebung isoliert ist, die sich außerhalb des Überzugs befindet, **dadurch gekennzeichnet, dass** die Dichtungseinrichtung enthält:
einen Klemmring (64, 90, 114), der zwischen dem ersten Ende und dem zweiten Ende der sterilen Kupplung angeordnet werden kann und mit dem Überzugsring in Eingriff kommt, um den Überzugsring an der Kupplung zwischen dem Klemmring und der Kupplung zusammenzudrücken.

2. Vorrichtung nach Anspruch 1, wobei die sterile Sperre enthält:
ein optisch transparentes Fenster (53, 73, 103), das quer auf dem Lichtweg angebracht ist.

3. Vorrichtung nach Anspruch 1, wobei die zweite Anbringungsstruktur enthält:
einen inneren Durchlass (55) zum Aufnehmen des sterilen Endoskops; und
eine Befestigungseinrichtung (58), die an der zweiten Anbringungsstruktur angebracht ist, um die zweite Anbringungsstruktur lösbar an dem sterilen Endoskop anzubringen.

4. Vorrichtung nach Anspruch 3, wobei die Befestigungseinrichtung (58) eine Gleit-Schnelltrenneinrichtung enthält.

5. Vorrichtung nach Anspruch 1, wobei die zweite Anbringungsswktur enthält:
eine quer angebrachte Befestigungseinrichtung (58, 81, 108) zum lösbaren Anbringen der zweiten Anbringungsstruktur an dem sterilen Endoskop.

6. Vorrichtung nach Anspruch 1, wobei die sterile Kupplung des Weiteren enthält:
einen integralen Eingriffsflansch (63); und
den Überzugsring (62), der an dem vorderen Ende des Überzugs zum lösbaren Eingriff mit dem Eingriffsflansch (63) und dem Klemmring (64) angebracht ist

7. Vorrichtung nach Anspruch 1, wobei der Klemmring des Weiteren enthält:
ein Dichtungselement (66, 68), das an einer Seite des Kiemmrings angebracht ist, um den Überzugsring (62) festzuhalten, wobei das Dichtungselement einen luftund flüssigkeitsdichten Verschluss zwischen dem optischen Verbinder und der sterilen Kupplung bildet.

8. Vorrichtung nach Anspruch 1, wobei der Klemmring des Weiteren enthält:
einen Flansch (92, 117) zum Festhalten des Überzugsrings (86, 112) in einer Nut (89, 118), die an der sterilen Kupplung (70, 100) ausgebildet ist, wobei der Flansch einen luft- und flüssigkeitsdichten Verschluss zwischen dem optischen Verbinder und der sterilen Kupplung bildet.

9. Vorrichtung nach Anspruch 1, die des Weiteren enthält:
einen sterilen, wegwerfbaren Stab (120), der an dem zweiten Ende der sterilen Kupplung angebracht werden kann, um die Kameraanordnung zu tragen, wenn sie an der sterilen Kupplung angebracht ist

10. Vorrichtung nach Anspruch 1, wobei:
die erste Anbringungssttuktur einen optischen Kupplungsteil (71) mit einem Innengewindeabschnitt (77) enthält und die zweite Anbtingungsstruktur eirten Endoskop-Kupplungsteil (72) mit Außengewinde (79) zum Eingriff mit dem Innengewindeabschnitt (77) enthält, um das vordere Ende des Überzugs dazwischen lösbar zu befestigen.

11. Vorrichtung nach Anspruch 10, wobei:
der Klemmring des Weiteren einen Flansch (92) enthält, der das vordere Ende des Überzugs an einer Endoskop-Fassung (74) des Endoskop-Kupplungsteils (72) festhält.

12. Vorrichtung nach Anspruch 1, wobei:
die erste Anbringungsstruktur eine optische Kupplungs-Fassung (115) und einen inneren Durchlass (116) enthält, der durch die optische Kupplungs-Fassung hindurch ausgebildet ist; und
die zweite Anbringungsstruktur eine Endoskop-Fassung (104) mit einem ringförmigen Flansch (106) zum gleitenden Eingriff mit dem inneren Durchlass (116) enthält, um das vordere Ende des Überzugs zwischen der optischen Kupplungs-Fassung (115) und der Endoskop-Fassung (104) lösbar zu befestigen.

13. Vorrichtung nach Anspruch 12, wobei:
die optische Kupplungs-Fassung (115) an dem Klemmring (114) angebracht ist.

14. Verfahren zum Verbinden einer nicht sterilen Kameraanordnung, die einen optischen Verbinder (OC) und eine Kamera (C) enthält, mit einem sterilen Endoskop (E), wobei verschiedene Endoskope beliebig ausgewechselt werden können, ohne die Sterilität einer chirurgischen Umgebung zu beeinträchtigen, und das Verfahren die Schritte des Bereitstellens einer sterilen Kupplung (50, 70, 100), die ein erstes Ende, das an der nicht sterilen Kameraanordnung angebracht werden kann, und ein zweites Ende aufweist, das an dem sterilen Endoskop angebracht werden kann, des Bereitstellens eines sterilen Überzugs (60, 84, 110), der ein offenes vorderes Ende aufweist, und elnen daran ausgebildeten Überzugsring (62, 86, 112) enthält und über dem ersten Ende der Kupplung angebracht werden kann, und ein hinteres Ende aufweist, das über die Kameraanordnung ausgedehnt werden kann, des Einführens des nicht sterilen optischen Verbinders (OC), der an der nicht sterilen Kamera (C) angebracht ist, in dem hinteren Ende des Überzugs (60, 84, 110), des Anbringens des nicht sterilen optischen Verbinders an dem ersten Ende der Kupplung und des Anbringens des sterilen Endoskops an dem zweiten Ende der Kupplung umfasst, **dadurch gekennzeichnet, dass** das Verfahren des Weiteren den folgenden Schritt umfasst:
Abdichten des vorderen Endes des Überzugs (60, 84, 110), um das erste Ende der Kupplung (50, 70, 100) herum mit einem Klemmring (64, 90, 114), der mit dem Überzugsring und der Kupplung in Eingriff gebracht werden kann, um so eine sterile Dichtung zu schaffen, wobei das erste Ende der Kupplung in dem Überzug abgedichtet ist und das zweite Ende der Kupplung zu der sterilen chirurgischen Umgebung hin freiliegt.

15. Verfahren nach Anspruch 14, das des Weiteren die folgenden Schritte enthält:
Tragen der sterilen Kupplung mit einem sterilen, wegwerfbaren Stab (120), der an dem zweiten Ende derselben angebracht ist, vor dem Anbringen des nicht sterilen optischen Verbinders (OC) an dem ersten Ende der Kupplung; und
Lösen des sterilen, wegwerbaren Stabs (120) von dem zweiten Ende der sterilen Kupplung vor dem Anbringen des sterilen Endoskops am zweiten Ende der Kupplung.

## Revendications

1. Appareil de couplage optique d'un endoscope stérile (E) à un ensemble à caméra stérile, comprenant un connecteur optique (OC) non stérile monté sur une caméra (UC) non stérile ayant des câbles libres, et destiné à entourer l'ensemble à caméra non stérile dans une enceinte stérile afin qu'un ensemble à caméra non stérile puisse être utilisé dans des conditions chirurgicales stériles, l'appareil comprenant un coupleur stérile (50, 70, 100), ayant une première extrémité qui comporte une première structure de montage (56, 71, 115) destinée à la connexion au connecteur optique non stérile (OC) et ayant une seconde extrémité qui comporte une seconde structure de montage (54, 72, 104) destinée à la connexion à l'endoscope stérile (E), le coupleur stérile ayant en outre un trajet optique disposé entre les première et seconde extrémités de celui-ci par lesquelles une image peut être transmise de l'endoscope stérile à la caméra non stérile, un barrage stérile (53, 73, 103) destiné à être positionné entre les première et seconde extrémités du coupleur stérile,
une jupe stérile (60, 84, 110) ayant une extrémité distale ouverte et comprenant un anneau de jupe (62, 86, 112) formé sur elle, la jupe stérile étant destinée à être placée sur la première extrémité du coupleur et ayant une extrémité proximale qui peut s'étendre sur le connecteur optique non stérile, la caméra non stérile et ses câbles libres, et un dispositif destiné à assurer l'étanchéité entre l'extrémité distale de la jupe entre la première et la seconde extrémité du coupleur stérile si bien que la première extrémité du coupleur est isolée de l'environnement chirurgical stérile placé à l'extérieur de la jupe, **caractérisé en ce que** le dispositif d'étanchéité comprend :
un anneau de blocage (64, 90, 114) destiné à être placé entre la première extrémité et la seconde extrémité du coupleur stérile et coopérant avec l'anneau de jupe pour comprimer l'anneau de jupe contre le coupleur entre l'anneau de blocage et le coupleur.

2. Appareil selon la revendication 1, dans lequel le barrage stérile comprend :
une fenêtre optiquement transparente (53, 73, 103) montée transversalement sur le trajet optique.

3. Appareil selon la revendication 1, dans lequel la seconde structure de montage comporte :
un passage interne (55) destiné à loger l'endoscope stérile, et
un dispositif de fixation (58) fixé à la seconde structure de montage pour la fixation temporaire de la seconde structure de montage à l'endoscope stérile.

4. Appareil selon la revendication 3, dans lequel le dispositif de fixation (58) comporte un organe coulissant de libération rapide.

5. Appareil selon la revendication 1, dans lequel la seconde structure de montage comprend :
un dispositif (58, 81, 108) de fixation monté transversalement et destiné à la fixation temporaire de la seconde structure de montage sur l'endoscope stérile.

6. Appareil selon la revendication 1, dans lequel le coupleur stérile comporte en outre :
un flasque solidaire de coopération (63),
l'anneau de jupe (62) étant fixé à l'extrémité distale de la jupe afin qu'il coopère temporairement avec le flasque de coopération (63) et l'anneau de blocage (64).

7. Appareil selon la revendication 1, dans lequel l'anneau de blocage comporte en outre :
un organe d'étanchéité (66, 68) fixé à un premier côté de l'anneau de blocage afin qu'il retienne l'anneau de jupe (62), l'organe d'étanchéité formant un joint étanche au liquide et hermétique entre le connecteur optique et le coupleur stérile.

8. Appareil selon la revendication 1, dans lequel l'anneau de blocage comporte en outre :
un flasque (92, 117) destiné à retenir l'anneau de jupe (86, 112) dans une gorge (89, 118) formée sur le coupleur stérile (70, 100), le flasque formant un joint étanche au liquide et hermétique entre le connecteur optique et le coupleur stérile.

9. Appareil selon la revendication 1, comprenant en outre :
une baguette jetable stérile (120) destinée à être fixée à la seconde extrémité de couplage stérile et destinée à former un support pour l'ensemble à caméra lorsqu'il est fixé au coupleur stérile.

10. Appareil selon la revendication 1, dans lequel :
la première structure de montage comprend une partie de coupleur optique (71) ayant une partie taraudée (77), et la seconde structure de montage comprend une partie de coupleur d'endoscope (72) ayant un filetage (79) destiné à coopérer avec le taraudage (77) pour la fixation temporaire de l'extrémité distale de la jupe en position intermédiaire.

11. Appareil selon la revendication 10, dans lequel :
l'anneau de blocage comporte en outre un flasque (92) qui retient l'extrémité distale de la jupe contre une monture (74) d'endoscope de la partie (72) de coupleur d'endoscope.

12. Appareil selon la revendication 1, dans lequel :
la première structure de montage comporte une monture (115) de coupleur optique et un passage interne (116) formé dans la monture de coupleur optique, et
la seconde structure de montage comprend une monture (104) d'endoscope ayant un flasque annulaire (106) destiné à coopérer par coulissement avec le passage interne (116) pour la fixation amovible de l'extrémité distale de la jupe entre la monture (115) du coupleur optique et la monture (104) de l'endoscope.

13. Appareil selon la revendication 12, dans lequel :
la monture (115) de coupleur optique est fixée à l'anneau de blocage (114).

14. Procédé de couplage d'un ensemble à caméra non stérile comprenant un connecteur optique (OC) et une caméra (C) sur un endoscope stérile (E), tel que différents endoscopes peuvent être échangés librement sans compromettre la stérilité de l'environnement chirurgical, le procédé comprenant des étapes de disposition d'un coupleur stérile (50, 70, 100) ayant une première extrémité destinée à être fixée à l'ensemble à caméra non stérile et une seconde extrémité destinée à être fixée à l'endoscope stérile, de disposition d'une jupe stérile (60, 84, 110) ayant une extrémité distale ouverte comprenant un anneau de jupe (62, 86, 112) formé sur la jupe, destinée à être disposée sur la première extrémité du coupleur et ayant une extrémité proximale sur l'ensemble à caméra, d'insertion du connecteur optique non stérile (OC) monté sur la caméra non stérile (C) à l'extrémité proximale de la jupe (60, 84, 110), de fixation du connecteur optique non stérile à la première extrémité du coupleur, et de fixation de l'endoscope stérile à la seconde extrémité du coupleur, **caractérisé en ce que** le procédé comporte en outre l'étape suivante :
la mise en coopération étanche de l'extrémité distale de la jupe (60, 84, 110) autour de la première extrémité du coupleur (50, 70, 100) à l'aide d'un anneau de blocage (64, 90, 114) destiné à coopérer avec l'anneau de jupe et le coupleur en formant ainsi un joint d'étanchéité stérile dans lequel la première extrémité du coupleur est mise en coopération étanche dans la jupe et la seconde extrémité du coupleur est exposée à l'environnement chirurgical stérile.

15. Procédé selon la revendication 14, comprenant en outre les étapes suivantes :
le support du coupleur stérile à l'aide d'une baguette jetable stérile (120) fixée à la seconde extrémité du coupleur avant fixation du connecteur optique non stérile (OC) à la première extrémité du coupleur, et
la séparation de la baguette jetable stérile (120) de la seconde extrémité du coupleur stérile avant la fixation de l'endoscope stérile à la seconde extrémité du coupleur.
